(19) Europäisches Patentamt
European Patent Office
Office européen des brevets

(11) Publication number: **0 166 937 B1**

(12) **EUROPEAN PATENT SPECIFICATION**

(45) Date of publication of patent specification: **28.08.91**

(51) Int. Cl.5: **C07D 233/22**, C07D 239/06, C07D 239/26, C07C 257/00, A61K 31/415, A61K 31/505, C07D 405/04

(21) Application number: **85106209.1**

(22) Date of filing: **21.05.85**

The file contains technical information submitted after the application was filed and not included in this specification

# (54) Adrenergic compounds.

(30) Priority: **06.06.84 US 617770**

(43) Date of publication of application:
**08.01.86 Bulletin 86/02**

(45) Publication of the grant of the patent:
**28.08.91 Bulletin 91/35**

(84) Designated Contracting States:
**BE CH DE FR GB IT LI NL SE**

(56) References cited:
**EP-A- 0 119 107**
**FR-A- 2 542 738**
**GB-A- 1 475 586**
**US-A- 2 948 724**

(73) Proprietor: **ABBOTT LABORATORIES**
**14th Street and Sheridan Road**
**North Chicago, Illinois 60064(US)**

(72) Inventor: **DeBernardis, John Francis**
**35 Burnett Avenue**
**Lake Villa, IL 60046(US)**
Inventor: **Basha, Fatima Zehra**
**232 Bayshore Drive**
**Lake Bluff, IL 60044(US)**

(74) Representative: **Modiano, Guido et al**
**MODIANO, JOSIF, PISANTY & STAUB**
**Modiano & Associati Via Meravigli, 16**
**I-20123 Milano(IT)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

Background of the Invention

This invention relates to novel adrenergic compounds useful in the treatment of hypertension, depression, nasal congestion, metabolic disorders (e.g. obesity) and migraine.

The adrenergic nervous system plays a major role in the innervation of heart, blood vessel and smooth muscle tissue. Compounds capable of interacting with receptor sites within the adrenergic nervous system can initiate a variety of physiological responses, including vasoconstriction, vasodilation, and increased or decreased heart rate (chronotropic), contractility (inotropic) and metabolic activity. In the past, various adrenergic compounds have been employed to affect these and other physiological responses. However, many adrenergic compounds do not possess significant selectivity to enable desirable interactions with adrenergic receptor sites. That is, these adrenergic compounds do not demonstrate a high degree of specificity for differing receptor types within the adrenergic nervous system in order to obtain a desired physiological response separate from other possible, and perhaps less desirable, responses of the system. It has now been determined that a new class of compounds, as herein defined, demonstrate an ability to selectively stimulate (agonists) or inhibit (antagonists) α-adrenergic receptors which are mainly distributed on the membranes of central and peripheral adrenergic neurons and on the tissues innervated thereby.

Through inhibitory interaction with the α-adrenergic receptor in the peripheral nervous system, one can modulate the function of adrenergic neurons and hemodynamic equilibrium which is therapeutically useful in a multitude of cardiovascular indications such as hypertension, congestive heart failure, and a variety of vascular spastic conditions. Stimulatory interaction with α-adrenergic receptors in the peripheral nervous system is therapeutically useful in situations where vascular constrictions such as nasal, otic or ophthalmic congestion and inflammation occur.

In the central nervous system, stimulatory - adrenergic receptor agonists are therapeutically useful for sedation, diuresis, and in treatment of addictive behavior and hypertension. The α-adrenergic antagonists in the central nervous system are useful in certain neurological and psychiatric disorders such as depression as well as for maintenance of cardiovascular equilibrium.

Disclosed in US Patent No. 2 948 724 are adrenergic compounds comprising halogenated derivatives of tetrahydro-1-naphthyl cyclic amidines. European Patent Application No. 0 119 107 discloses bicyclo [4.2.o] octatriene-1,3,5 derivatives as adrenergic compounds.

The therapeutic usefulness of the compounds of this invention stems from their selectivity for adrenergic receptor subtypes and their selective modulation of the adrenergic function in different tissues or organs.

Disclosure of the Invention

The present invention provides compounds represented by the formula (I)

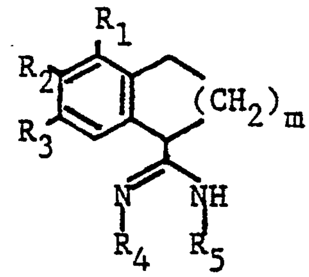

wherein m is 0, 1 or 2; $R_1$, $R_2$, $R_3$ are taken from the group consisting of hydrogen, hydroxy, loweralkyl, loweralkoxy or halo, provided that $R_1$ , $R_2$, $R_3$ cannot simultaneously be hydrogen and provided further that when one of $R_1$, $R_2$, $R_3$ is halo, the other two cannot simultaneously be hydrogen; $R_1$ and $R_2$ or $R_2$ and $R_3$ taken together can form a methylenedioxy bridge; and $R_4$ and $R_5$ are hydrogen or taken together form a closed ring of the formula

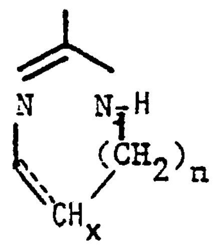

wherein n is 0 or 1, x is 1 or 2 and the dashed line represents a second bond when x is 1, provided that when m=0, x=2, n=1 and $R_1$ and/or $R_2$ is methoxy, the others among $R_1$, $R_2$ and $R_3$ cannot simultaneously be hydrogen, and the pharmaceutically acceptable salts thereof.

As used herein, the term "loweralkoxy" refers to alkoxy groups containing from 1 to 6 carbon atoms in straight or branched chains with or without double bonds, i.e., methoxy, ethoxy, propoxy, isopropoxy, butoxy, allyloxy, etc.

The term "loweralkyl" means straight or branched chain saturated hydrocarbon radicals having 1 to 6 carbon atoms, such as methyl, ethyl,

n-propyl, iso-propyl, n-butyl, s-butyl, and t-butyl.

The term "arylalkyl" refers to an aryl group substituted with a loweralkyl as defined above.

The term "pharmaceutically acceptable salts" refers to the pharmaceutically acceptable, relatively nontoxic, inorganic or organic acid addition salts of the compounds of this invention. These salts can be prepared in situ during the final isolation and purification of the compounds, or by separately reacting the free base with a suitable organic or inorganic acid. Repesentative salts include the hydrochloride, hydrobromide, sulfate, phosphate, nitrate, bisulfate, acetate, oxalate, valerate, oleate, palmitrate, stearate, laurate, borate, benzoate, lactate, phosphate, tosylate, citrate, maleate, fumarate, succinate, tartrate, napsylate and the like. It will be apparent to those skilled in the art that, depending upon the number of available amino groups for salt formation, the salt of this invention can be per-N-salts.

The foregoing may be better understood in connection with the following examples:

Example 1

1-Cyano-5,6-dimethoxy-(3,4-dihydronaphthalene)

5,6-dimethoxytetralone (20 g) was dissolved in benzene (20 ml) and trimethylsilyl cyanide (16 ml). A catalytic amount of aluminum chloride was added and the reaction heated at 70° C under nitrogen for 30 minutes. The volatiles were removed in vacuo and methanol (20 ml) added. Gaseous hydrochloric acid was bubbled through the methanol solution for 30 minutes resulting in precipitation of a solid. Stirring was continued for 1 hour, then the mixture was filtered and dried affording 19.9 g. of product (96% yield); m.p. 138-9° C.

Example 2

1-Cyano-5,6-dimethoxy-(1,2,3,4-tetrahydronaphthalene)

The product from Example 1 (10.5 g) was refluxed for 2.5 hours in ethanol (200 ml) containing sodium borohydride (6 g). The reaction was cooled to room temperature and evaporated to dryness. Aqueous hydrochloric acid (50 ml) was carefully added followed by extraction with dichloromethane. The organic layer was separated, dried and evaporated affording 8.4 g. of the desired product (79% yield); m.p. 50-1° C.

Example 3

Ethyl 5,6-dimethoxy-(1,2,3,4-tetrahydro-naphthalene)-1-carboximidate

1-Cyano-5,6-dimethoxy-(1,2,3,4-tetrahydronaphthalene (15 g) was dissolved in 140 ml diethylether and then ethanol (70 ml) added. The solution was cooled to 0° C. and hydrochloric acid bubbled through for 2 hours. The reaction was allowed to warm to room temperature, stoppered tightly and allowed to stand at room temperature overnight. The solvent was evaporated, then acetonitrile added followed again by evaporation of the volatiles. The oily residue was taken up in acetonitrile (25 ml) and the product was precipitated by addition of diethylether (200 ml) to this solution. The product was filtered and air dried giving 14.5 g.; m.p. 137-8(d).

Example 4

5',6'-Dimethoxy-2 -(1',2',3',4'-tetrahydro-1'-naphthyl)-imidazoline HCl

The product from Example 3 (2.0 9) was dissolved in ethanol (25 ml) and cooled to 0° C. Ethylene diamine (1.8 ml) was added dropwise to this solution and upon complete addition stirring was continued for 1 hour at 0° C. The reaction was then allowed to warm to room temperature overnight. The solvent was removed in vacuo giving an oil. Water (50 ml) was added followed by a diethylether extraction (70 ml), then a dichloromethane extraction (100 ml). The organic extracts were separated, combined, dried and evaporated giving an oil which solidified upon standing (1.7 g). This material was dissolved in ether (5 ml) and added to ethereal hydrochloric acid (15 ml) and the resulting salt filtered and dried; m.p. 245-7° C.

Example 5

5',6'-Dihydoxy-2 -(1',2',3',4'-tetrahydro-1'-naphthyl)-imidazoline HBr

The product from Example 4 (1.7 g) was dissolved in dichloromethane (50 ml) and cooled to -78° C under nitrogen. Borontribromide (2.4 ml) in 10 ml dichloromethane was added dropwise to this solution, and upon complete addition the reaction was stirred at room temperature for 2 hours. This was then cooled to -78° C and excess borontribromide quenched by dropwise addition of methanol (25 ml). Upon complete addition of the methanol, the mixture was allowed to warm to room temperature and stirred for 1 hour. The solution was evaporated in vacuo affording a solid; m.p. 268-70° C.

Example 6

1-Cyano-5,6-methylenedioxy-(3,4-dihydronaphthylene)

Utilizing the procedure of Example 1 but replacing the 5,6-dimethoxy-1-tetralone with 5,6-methylenedioxy-1-tetralone afforded the desired product; m.p. 82-83° C.

Example 7

1-Cyano-5,6-methylenedioxy-(1,2,3,4-tetrahydronaphthalene)

The product from Example 6 was reduced using the procedure of Example 2 to afford the product in 98% yield.

Example 8

Ethyl 5,6-methylenedioxy-(1,2,3,4-tetrahydro-naphthylene)-1-carboximidate

The product from Example 7 (3.8 g) was dissolved in absolute ethanol (30 ml) and diethylether (60 ml) and the imino ether formed as in Example 3 affording the product in 87% yield; m.p. 185-7°.

Example 9

5',6'-Methylenedioxy-2 -(1',2',3',4'-tetrahydro-1'-naphthyl)imidazoline HCl

Using the product from Example 8 and the procedure of Example 4 afforded the desired product as the monohydrate; m.p. 264-65° C.

Example 10

1-Cyano-6,7-dimethoxy-(1,2,3,4-tetrahydronaphthalene)

Starting with 1-cyano-6,7-dimethoxy-(3,4-dihydronaphthylene) (17.7 g) and using the procedure of Example 2 afforded the desired compound in 98% yield as an oil.

Example 11

Ethyl, 6,7-dimethoxy(1,2,3,4-tetrahydro-naphthylene)-1-carboximidate

The product from Example 10 (15 g) was reacted as in Example 3 to afford the desired product (18.8 g) in 92% yield; m.p. 179-82° C.

Example 12

6',7,-Dimethoxy-2 -(1',2',3',4'-tetrahydro-1'-naphthyl)imidazoline HCl

The product from Example 11 (2.5 g) was dissolved in ethanol (30 ml) and reacted with ethylenediamine (1.2 ml) as in Example 4 giving the desired product; m.p. 253-4° C.

Example 13

6',7,-Dihydroxy-2 -(1',2',3',4'-tetrahydro-1'-naphthyl)imidazoline HBr

The product from Example 12 (1.2 g) was reacted with borontribromide (1.5 ml) as in Example 5 affording the product; m.p. 260-61° C.

Example 14

1-Cyano-5-methoxy-(3,4-dihydronaphthalene)

Trimethylsilyl cyanide (28.2 g) and a catalytic amount of aluminum trichloride was added to a toluene solution of 5-methoxy-1-tetralone (25 g) and the reaction heated for 2 hours. The solution was evaporated to dryness. Pyridine (100 ml) was then added, followed by dropwise addition of phosphorous oxychloride (40 ml). Upon complete addition the reaction was heated to 120° C for 2 hours. The reaction was cooled and poured onto ice containing aqueous hydrochloric acid. The mixture was extracted with ether (100 ml). The organic layer was separated, dried, filtered and evaporated affording the desired product as an oil which solidified upon standing.

Example 15

1-Cyano-5-methoxy-(1,2,3,4-tetrahydronaphthalene)

The product from Example 14 (11.4 g) was refluxed for 2.5 hours in ethanol (250 ml) containing sodium borohydride (3.0 g). The reaction was cooled to room temperature and evaporated to dryness. Aqueous hydrochloric acid (50 ml) was carefully added followed by extraction with dichloromethane. The organic layer was separated, dried and evaporated affording the product (9.2 g).

Example 16

Ethyl-5-methoxy(1,2,3,4-tetrahydro-naphthalene)-1-carboximidate

The product from Example 15 (4.7 g) was dissolved in ethanol (50 ml) and ether (50 ml). The solution was cooled to 0° C and hydrochloric acid (g) bubbled through this for 2 hours. The reaction

was allowed to warm to room temperature, stoppered tightly and allowed to stand at room temperature overnight. The solvent was evaporated. Acetonitrile was then added, followed again by evaporation of the volatiles. The oily residue was taken up in acetonitrile (25 ml) and the product precipitated by addition of diethylether (200 ml). The product was filtered and air dried affording the desired product.

Example 17

5'-Methoxy-2 -(1',2',3',4'-tetrahydro-1'naphthyl) imidazoline .HCl

The product from Example 16 (5.9 g) was dissolved in ethanol (50 ml) and cooled to 0° C. Ethylene diamine (5.4 ml) was added dropwise to this solution and upon complete addition, stirring was continued for 1 hour at 0° C and then at room temperature overnight. The solvent was evaporated to dryness. The residue was taken up in ether. Ethereal hydrochloric acid was then added. The hydrochloric acid salt was filtered and dried; m.p. 198-99° C.

Example 18

5'-Hydroxy-2 -(1',2',3',4'-tetrahydro-1'-naphthyl)-imidazoline HBr

The product from Example 17 (1.6 g) was dissolved in methylene chloride (25 ml) and cooled to -78° C under nitrogen. Boron tribromide (2.7 ml) in 5 ml methylene chloride was added dropwise to this solution and upon complete addition, the reaction was stirred at room temperature for 2 hours. After this time, the reaction was cooled to -78° C and quenched by the dropwise addition of methanol (25 ml). The solution was evaporated to dryness and the residue crystallized from an ethanol-ether mixture affording the product; m.p. 229-30° C.

Example 19

6'-Methoxy-2 -(1',2',3',4'-tetrahydro-1'-naphthyl)-imidazoline HCl

1-Cyano-6-methoxy-(1,2,3,4-tetrahydronaphthalene) (5.25 g) and the mono paratoluene sulfonate salt of ethylenediamine (20.9 g) was heated to 250° C in a flask under nitrogen, for ca. 4 minutes. The reaction was cooled to room temperature and a solid mass resulted. This solid was dissolved in 6N hydrochloric acid (ca. 250 ml) and extracted with diethylether (3 x 150 ml). The aqueous layer was separated and made basic with sodium hydroxide, followed by methylene chloride extraction (3 x 100 ml). The organic layers were combined, dried, filtered and evaporated to dryness giving an oily residue. This residue was dissolved in ethanol (25 ml) and ethereal hydrochloric acid added. Evaporation to dryness gave a glass which upon trituration with acetonitrile afforded a solid; m.p. 182-184° C.

Example 20

6'-Hydroxy-2 -(1',2',3',4',-tetrahydro-1'-naphthyl)-imidazoline HBr

The product from Example 19 (1.2 g) was suspended in methylene chloride (20 ml) and cooled to -78° C. Boron tribromide (2.5 ml) in methylene chloride (5 ml) was added dropwise. Upon complete addition, reaction was allowed to warm to room temperature and stirred for 2 hours. The reaction was then cooled to -78° C and quenched by the dropwise addition of methanol (25 ml), followed by evaporation to dryness. The resulting solid was crystallized from ethanol/acetonitrile mixture affording the desired product; m.p. 254-55° C.

Example 21

7'-Methoxy-2 -(1',2',3',4'-tetrahydro-1'-naphthyl)-imidazoline HCl

Ethyl-7-methoxy(1,2,3,4-tetrahydronaphthalene)-1-carboximidate (6 g) was dissolved in 50 ml ethanol and reaction cooled to 0° C, followed by the dropwise addition of ethylenediamine (5.4 ml). The reaction was stirred at room temperature overnight, then evaporated to dryness affording a wax. The wax was suspended in water and extracted with diethylether. The ether solubles were washed with brine, separated and dried. Evaporation gave a glass, to which methanolic hydrochloric acid was added. The solution was evaporated to dryness and diethylether and a few drops of acetonitrile added. The light yellow solid was filtered and dried; m.p. 162-63° C.

Example 22

7'-Hydroxy-2 -(1',2',3',4'-tetrahydro-1'-naphthyl)-imidazoline HBr

The product from Example 21 (0.68g) was suspended in methylene chloride (20 ml) and then cooled to -10° C. Boron tribromide (1.4 ml) in methylene chloride (5 ml) was added dropwise. After the addition, the reaction was allowed to warm to room temperature and stirred for 2 hours.

The reaction was cooled to -78° C and quenched with methanol. Evaporation of the solvents gave a white solid which was slurried in ether, then filtered; m.p. 255-56° C.

Example 23

5'-Methoxy-2 -(1'-indanyl)imidazoline HCl

1-Cyano-5-methoxy indane was dissolved in 30 ml ethanol and 30 ml diethylether, and gassed with dry hydrochloric acid for 25 minutes and then allowed to stand in a stoppered flask at room temperature overnight. The solvents were removed, then ethylenediamine (3.0 g) and ethanol (50 ml) were added, and the reaction heated for 30 Minutes and evaporated to dryness. The residue was taken up in ethanol (10 ml) and ethereal hydrochloric acid added. The solution was evaporated to dryness affording the product as a glass.

Example 24

5'-Hydroxy-2 -(1'-indanyl)imidazoline HBr

The product from Example 23 (1.5 g) was dissolved in methylene chloride (20 ml), then cooled to -78° C followed by the dropwise addition of $BBr_3$ in 5 ml methylene chloride. The reaction was allowed to warm to room temperature for 2 hours, then cooled to -78° C and quenched with methanolic hydrochloric acid (20 ml). Evaporation of the solvents afforded a solid which was crystallized from methanol; m.p. 283-84° C.

Example 25

Ethyl-4,5-dimethoxy-indane-1-carboximidate

1-Cyano-4,5-dimethoxy-(2,3-dihydroindane) (4.5 g), ethanol (50 ml) and diethylether (50 ml) were combined, then cooled to -78° C, followed by gassing with dry hydrochloric acid for 30 minutes. After this time, the reaction was allowed to stand at room temperature for 22 hours, and the solvents removed, affording a solid.

Example 26

4',5'-Dimethoxy-2 -(1'indanyl)-imidazoline HCl

The product from Example 25 (4.3 g) was dissolved in ethanol (50 ml) and ethylenediamine (8.5 ml) and the reaction was stirred at room temperature overnight, followed by heating for 30 minutes. Evaporation of the solvents afforded an oil, which solidified upon standing. This was crystallized from acetonitrile affording a white solid. This was dissolved in ethanol (10 ml) and ethereal hydrochloric acid added. The resulting hydrochloric acid salt was recrystallized from acetonitrile affording the product; m.p. 226-227° C.

Example 27

4',5'-Dihydroxy-2 -(1'-indanyl)imidazoline HBr

The product from Example 26 (0.6 g) was dissolved in methylene chloride (20 ml) and cooled to -78° C. Boron tribromide (2 ml) in methylene chloride (5 ml) was then added dropwise. The reaction was allowed to warm to room temperature overnight, then quenched with methanol (25 ml). Evaporation afforded a grayish solid, which was washed with ether to give a white solid; m.p. 239-240° C.

Example 28

5',6,-Dimethoxy-2 -(1',2',3',4'-tetrahydro-1'-naphthyl)-pyrimidine HCl

To an ethanol (20 ml) solution of ethyl-5,6-dimethoxy-(1,2,3,4-tetrahydronaphthylene)-1-carboximidate was added 1,3-diaminopropane (1.25 ml) and the reaction refluxed one hour. The solution was evaporated to dryness and aqueous potassium hydroxide (50 ml) added, followed by methylene chloride (200 ml) extraction. The organic layer was separated, dried, filtered and then methanolic hydrochloric acid added. The solution was evaporated to dryness. Methylene chloride was then added. Diethylether was added until the product began to crystallize from solution; m.p. 208-9° C.

Example 29

5',6'-Dihydroxy-2 -(1',2',3',4'-tetrahydro-1'-naphthyl)-pyrimidine HBr

The product from Example 28 (1.0 g) was dissolved in methylene chloride (30 ml) and cooled to -78° C. Then boron tribromide (1.2 ml) in methylene chloride (5 ml) was added dropwise. Upon complete addition, the reaction was allowed to warm to room temperature, then stirred for 1 hour. The reaction was cooled to -78° C and quenched with methanol (20 ml). Evaporation afforded a solid which was crystallized from a methanol/ether mixture giving the product; m.p. 221-3° C.

Example 30

5',6'-dimethoxy-2 -(1',2',3',4'-tetrahydro-1'-naphthyl)-imidazole HCl

Ethyl-5,6-dimethoxy-(1,2,3,4-tetrahydro-naphthyl)-1-carboximidate (3 g) was dissolved in ethanol (30 ml), then aminoacetaldehyde diethylacetal (1.8 ml) was added and the solution refluxed for 16 hours. The solvent was evaporated and 4N hydrochloric acid (50 ml) added, followed by heating at 60° C for 24 hours. The reaction was cooled, 45% potassium hydroxide added and solids precipitated. The solid was filtered, washed with water and dried. Methylene chloride was added to dissolve the solid and then ethereal hydrochloric acid afforded the product; m.p. 168-69° C.

Example 31

5',6-Dihydroxy-2 -(1',2',3',4'-tetrahydro-1'-naphthyl)-imidazole .HBr

The product from Example 30 (1.03 g) was dissolved in methylene chloride (30 ml), then cooled to -78° C and boron tribromide (1.3 ml) in methylene chloride (5 ml) added dropwise. The reaction was then allowed to reach room temperature. After 30 minutes a solid precipitated. The reaction was cooled to -78° C and quenched with methanol (30 ml). An initial solution resulted followed by precipitation of a solid which was filtered affording the product; m.p. 72-6° C.

Example 32

5,6-Dimethoxy-(1,2,3,4-tetrahydronaphthyl)-1-carboxamidine .HCl

Ethyl-5,6-dimethoxy-(1,2,3,4-tetrahydronaphthyl)-1-carboximidate (1.0 g) in ethanol (30 ml) was refluxed overnight with ammonium hydroxide (10 ml). The solvents were then removed under vacuum. The solid was converted to the hydrochloric acid salt affording the desired product.

Example 33

5,6-Dihydroxy-(1,2,3,4-tetrahydro-naphthyl)-1-carboxamidine .HBr

The product from Example 32 (.65 g) was dissolved in methylene chloride (30 ml) and the boron tribromide (1.1 ml) in methylene chloride (5 ml) added dropwise. The procedure of Example 5 was then followed, affording the desired product; m.p. 270-72° C.

Example 34

4',5'-Methylenedioxy-2 -(1'-indanyl)imidazoline HCl

Utilizing the procedure from Example 4 with 4,5-methylenedioxy indanyl-1-carboximidate (2.1 g) in ethanol (15 ml) and ethylenediamine (1 ml) afforded the desired product; m.p. 221-23° C.

Example 35

5'-Methoxy-6'-hydroxy-2 -(1',2',3',4'-tetrahydro-1'-naphthyl)imidazoline HCl

1-cyano-5-methoxy-6-hydroxy-(1,2,3,4-tetrahydro-naphthalene (.9 g) was dissolved in methanol (10 ml), diethylether (30 ml) and methylene chloride (30 ml) and cooled in an ice/isopropanol bath. The solution was then gassed with dry hydrogen chloride for 30 minutes, stoppered tightly, and allowed to stand overnight at room temperature.

The solvent was removed and the resulting solid taken up in methanol (20 ml) followed by the addition of ethylenediamine (.75 ml). The solution was refluxed for 3 hours, then stripped to dryness. The residue was dissolved in ethanol (5 ml). Then ethereal hydrochloric acid was added. The resulting hydrochloride salt was filtered and recrystallized from a mixture of ethanol and diethylether affording the product; m.p. 165-66° C.

Example 36

5'-hydroxy-6'-methoxy-2 -(1',2',3',4'-tetrahydro-1'-naphthyl)imidazoline HCl

Utilizing the procedure of Example 35, with 1-cyano-5-benzyloxy-6-methoxy-(1,2,3,4-tetrahydronaphthalene) (1.5 g) and ethylenediamine (.97 ml) gave the desired product; m.p. 258-59° C.

Example 37

6'-hydroxy-7'-methoxy-2 -(1',2',3',4'-tetrahydro-1'-naphthyl)imidazoline HCl

Using the procedure from Example 35 with 1-cyano-6-benzyloxy-7-methoxy-(1,2,3,4-tetrahydro-naphthylene) (2.4 g) and ethylenediamine (1.06 ml) afforded the desired product; m.p. 295-96° C.

Example 38

6'-methoxy-7'-hydroxy-2 -(1',2',3',4'-tetrahydro-1'-naphthyl)imidazoline HCl

Using the procedure from Example 35 with 1-cyano-6-methoxy-7-hyrdoxy-(1,2,3,4-tetrahydronaphthylene (2.4 g) and ethylenediamine

(2 ml) gave the compound; m.p. 243-44° C.

Example 39

5'-bromo-6'-methoxy-2 -(1',2',3',4'-tetrahydro-1'-naphthyl)imidazoline HCl

Using the procedure from Example 35 with 1-cyano-5-bromo-6-methoxy-(1,2,3,4-tetrahydronaphthylene) (1.5 g) and ethylenediamine (.97 ml) afforded the desired product; m.p. 273-74° C.

Example 40

5'-bromo-6'-hydroxy-2 -(1',2',3',4'-tetrahydro-1'-naphthyl)imidazoline HBr

Using the procedure of Example 5 with the product from Example 39 (.65 g) afforded the desired compound; m.p. 253-54° C.

Example 41

Methyl-3,4-dimethoxy-benzocyclobutane-1-carboximidate

Hydrogen chloride gas was bubbled into a 0° C solution of methanol (10 ml) diethylether (50 ml), and methylenechloride (50 ml) containing 1-cyano-3,4-dimethoxy-benzocyclobutane (5 g). After 15 minutes the flask was stoppered and kept in the refrigerator overnight. The solvents were evaporated and the residue triturated with $CH_2Cl_2$ (ca. 100 ml) affording a white solid; m.p. 207-8° C.

Example 42

3',4'-Dimethoxy-2 -(1'-benzocyclobutane)-imidazoline HCl

An ethanol (40 ml) suspension of the product from Example 41 was treated with ethylenediamine (2 ml). The reaction was stirred at room temperature for 2.5 hours. The solvent was removed and the oily residue partitioned between water and methylene chloride. The organic layer was separated, dried (magnesium sulfate), filtered, and evaporated, affording the amine. This was taken up in isopropyl alcohol (50 ml) and then isopropyl alcohol (3 ml) saturated with hydrogen chloride gas was added. The resulting hydrochloride salt was filtered and air dried; m.p. 214-15° C.

Example 43

3',4'-Dihydroxy-2 -(1'-benzocyclobutane)-imidazoline HBr

Using the procedure of Example 5 and the product from Example 42 gave the product which was recrystallized from ethanol; m.p. 225° C (d).

Example 44

5',7'-Dimethoxy-2 -(1',2',3',4'-tetrahydro-1'-naphthyl)imidazoline HCl

Utilizing the procedure from Example 35 with 1-cyano-5,7-dimethoxy-1,2,3,4-tetrahydronaphthalene (2.0 g.) and ethylenediamine (1.5 ml.) gave the compound; m.p. 173-174° C.

Example 45

5',7'-Dihydroxy-2 -(1',2',3',4'-tetrahydro-1'-naphthyl)imidazoline HBr

Utilizing the procedure from Example 5 with the product from Example 44 (.82 g.) afforded the desired compound; m.p. 263-64° C.

Example 46

5',6',7',Trimethoxy-2 -(1',2',3',4'-tetrahydro-1'-naphthyl)imidazoline HCl

Utilizing the procedure from Example 35 with 1-cyano-5,6,7-trimethoxy-1,2,3,4-tetrahydronaphthalene (2.7 g.) and ethylenediamine (1.06 ml.) gave the compound; m.p. 185-86° C.

Example 47

5',6',7'-Trihydroxy-2 -(1',2',3',4'-tetrahydro-1'-naphthyl)imidazoline HBr

Utilizing the procedure from Example 5 with the product from Example 46 (1.0 g.) gave the compound; m.p. 252-53° C.

Example 48

5'-Allyloxy-2 -(1',2',3',4'-tetrahydro-1'-naphthyl)-imidazoline HCl

Utilizing the procedure of Example 35 with 1-cyano-5-allyloxy-1,2,3,4-tetrahydronaphthalene (2.8 g.) and ethylenediamine (2.4 ml.) gave the compound; m.p. 184-85° C.

The selectivity of the compounds can be demonstrated in vitro by testing the compounds on isolated tissues such as stimulated rabbit aorta strips and by radioligand binding studies. The therapeutic activity of the compounds can be demonstrated in vivo by the compounds ability to affect arterial blood pressure and/or heart rate in various

experimental animals such as the spontaneously hypertensive (SH) rat.

In the latter test, a group of SH male rats are trained to be restrained in a wire mesh cylinder in a warming box, at least two training cycles being conducted before testing. The rats are warmed for about one-half hour period to blood pressure measurement, the warming box being maintained at a constant temperature of 36°C. An occluding cuff attached to a programmed sphymomanometer is placed near the base of the tail of each rat and the pressure in the cuff is increased automatically from 0 to 33000 Pa at a rate of 1300 Pa per second. The total time for each cycle of inflation and deflation of the cuff is 50 seconds and the interval between cycles is one minute. A photocell is placed distal to the cuff to record the pulses due to forward motion of blood flow with each heart beat. As the pressure in the cuff increases, the pulse disappears completely at a point where cuff pressure equals or exceeds the arterial blood pressure and it reappears during deflation at approximately the same pressure. Five interference free signals for deflation are recorded for each rat. Rats with a blood pressure of 24000 Pa or more during the control period are used in the study. Blood pressure and heart rate readings are recorded on a polygraph at various intervals after administration of the test compound. When tested in accordance with the foregoing procedure, the preferred compounds of the invention have decreased the arterial blood pressure and/or heart rate of rats of the group.

The compounds of the invention can be administered in any effective pharmaceutically acceptable form to warm blooded animals, e.g., in oral, parenteral or infusable dosage forms, or as a buccal or nasal spray. Suitable parenteral routes of administration include, for example, intramuscular, intravenous, intraperitoneal or subcutaneous administration of the compounds.

In addition to the active compounds, compositions according to this invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or other sterile injectable medium, immediately before use.

Solid dosage forms for oral administration include capsules, tablets, pills, powders and granules. In such solid dosage forms, the active compound may be admixed with at least one inert diluent such as sucrose, lactose or starch. Such dosage forms may also comprise, as is normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositons may also comprise adjuvants, such as wetting agents, emulsifying and suspending agents, and sweetening, flavoring and perfuming agents.

Actual dosage levels of active ingredient in the compositions of the invention may be varied so as to obtain an amount of active ingredient effective to obtain a desired therapeutic response for a particular composition and method of administration. The selected dosage level therefore depends upon the desired therapeutic effect, on the route of administration, on the desired duration of treatment and other factors. Generally, dosage levels of about 0.1 to about 200, more preferably about 0.5 to about 150 and most preferably about 1 to about 125 mg of active ingredient per kg of body weight per day are administered orally to a mammalian patient suffering from hypertension. If desired, the daily dose may be divided into multiple doses for administration, e.g., two to four separate doses per day.

The foregoing is merely illustrative of the invention and is not intended to limit the invention to the disclosed compounds.

## Claims

**1.** A compound of the formula

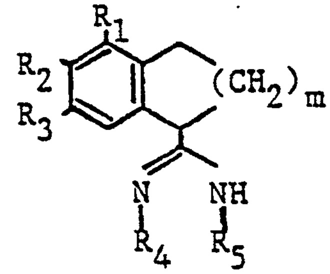

wherein m is 0, 1 or 2; $R_1$, $R_2$, $R_3$ are taken from the group consisting of hydrogen, hydroxy, $C_1$ to $C_6$ alkyl, $C_1$ to $C_6$ alkoxy or

halo, provided that $R_1$, $R_2$, $R_3$ cannot simultaneously be hydrogen and provided that when one of $R_1$, $R_2$, $R_3$ is halo, the other two cannot simultaneously be hydrogen; and $R_1$ and $R_2$ or $R_2$ and $R_3$ taken together can form a methylenedioxy bridge; and $R_4$ and $R_5$ are hydrogen or taken together form a closed ring of the formula

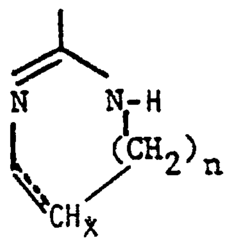

wherein n is 0 or 1, x is 1 or 2 and the dashed line representing a second bond when x is 1, provided that when m=0, x=2, n=1 and $R_1$ and/or $R_2$ is methoxy, the others among $R_1$, $R_2$ and $R_3$ cannot simultaneously be hydrogen, and the pharmaceutically acceptable salts thereof.

**2.** The compound in accordance with Claim 1 wherein m is 1 and n is 1.

**3.** The compound in accordance with Claim 1 wherein m is 2 and n is 1.

**4.** The compound in accordance with Claim 1 wherein $R_1$, $R_2$ and $R_3$ are taken from the group consisting of hydrogen, hydroxy or methoxy.

**5.** The compound in accordance with Claim 1 wherein $R_1$ and $R_2$ are taken together to form a methylenedioxy bridge.

**6.** The compound in accordance with Claim 1 wherein $R_1$ and $R_2$ are each hydroxy.

**7.** The compound in accordance with Claim 3 wherein $R_1$ and $R_2$ are each hydroxy.

**8.** The compound in accordance with Claim 7 wherein $R_3$ is hydrogen.

**9.** The compound in accordance with Claim 1 wherein $R_1$ and $R_3$ are each methoxy.

**10.** The compound in accordance with Claim 9 wherein $R_2$ is hydrogen.

**11.** The compound 5', 6'- dimethoxy -2- (1', 2', 3', 4'-tetrahydro - 1'- naphthyl)- pyrimidine, and the pharmaceutically acceptable salts thereof.

**12.** The compound 5', 6'- dihydroxy - 2 - (1', 2', 3', 4'-tetrahydro - 1' - naphthyl) - pyrimidine, and the pharmaceutically acceptable salts thereof.

## Revendications

**1.** composé de formule

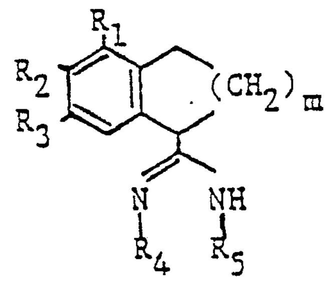

dans laquelle m est 0, 1 ou 2 ; $R_1$, $R_2$ et $R_3$ sont choisis parmi un hydrogène, un hydroxy, un alkyle en $c_1$ à $c_6$, un alcoxy en $c_1$ à $c_6$ ou un halogéno, sous réserve que $R_1$, $R_2$ et $R_3$ ne représentent pas simultanément un hydrogène et sous réserve de plus que, lorsque un de $R_1$, $R_2$ et $R_3$ est un halogéno, les deux autres ne peuvent pas être simultanément un hydrogène ; $R_1$ et $R_2$ ou $R_2$ et $R_3$ peuvent former ensemble un pont méthylènedioxy ; et $R_4$ et $R_5$ sont un hydrogène ou forment ensemble un cycle fermé de formule

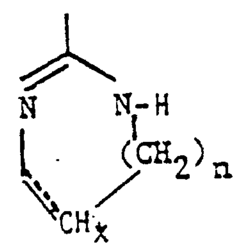

dans laquelle n est 0 ou 1, x est 1 ou 2, et le pointillé représente une seconde liaison lorsque x est 1, sous réserve que, lorsque m = 0, x = 2, n = 1 et $R_1$ et/ou $R_2$ est un méthoxy, les autres $R_1$, $R_2$ et $R_3$ ne peuvent pas simultanément être un hydrogène, et ses sels pharmaceutiquement acceptables.

**2.** Composé selon la revendication 1, dans lequel m est 1 et n est 1.

**3.** Composé selon la revendication 1, dans lequel m est 2 et n est 1.

4. Composé selon la revendication 1, dans lequel $R_1$, $R_2$ et $R_3$ sont choisis parmi un hydrogène, un hydroxy ou un méthoxy.

5. Composé selon la revendication 1, dans lequel $R_1$ et $R_2$ forment ensemble un pont méthylènedioxy.

6. Composé selon la revendication 1, dans lequel $R_1$ et $R_2$ sont chacun un hydroxy.

7. Composé selon la revendication 3, dans lequel $R_1$ et $R_2$ sont chacun un hydroxy.

8. Composé selon la revendication 7, dans lequel $R_3$ est un hydrogène.

9. Composé selon la revendication 1, dans lequel $R_1$ et $R_3$ sont chacun un méthoxy.

10. Composé selon la revendication 9, dans lequel $R_2$ est un hydrogène.

11. Le composé 5',6'-diméthoxy-2-(1',2',3',4'-tétrahydro-1'-naphtyl)pyrimidine et ses sels pharmaceutiquement acceptables.

12. Le composé 5',6'-dihydroxy-2-(1',2',3',4'-tétrahydro-1'-naphtyl)pyrimidine et ses sels pharmaceutiquement acceptables.

## Patentansprüche

1. Verbindung der Formel

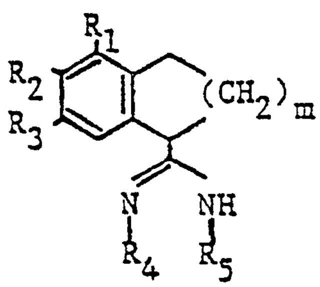

worin m 0, 1 oder 2 ist, $R_1$, $R_2$, $R_3$ aus der aus Wasserstoff, Hydroxy, $C_1$-$C_6$-Alkyl, $C_1$-$C_6$-Alkoxy oder Halogen bestehenden Gruppe genommen werden, mit der Maßgabe, daß $R_1$, $R_2$, $R_3$ nicht gleichzeitig Wasserstoff sein können und daß wenn eines von $R_1$, $R_2$, $R_3$ Halogen ist, die anderen beiden nicht gleichzeitig Wasserstoff sein können, und $R_1$ und $R_2$ oder $R_2$ und $R_3$ zusammengenommen eine Methylendioxybrücke bilden können; und $R_4$ und $R_5$ Wasserstoff sind oder zusammengenommen einen geschlossenen Ring der Formel bilden

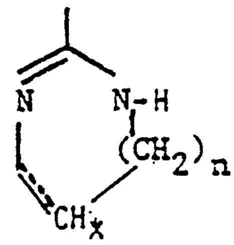

worin n 0 oder 1 ist, x 1 oder 2 ist und die gestrichelte Linie eine zweite Bindung darstellt, wenn x 1 ist, mit der Maßgabe, daß wenn $m=0$, $x=2$, $n=1$ und $R_1$ und/oder $R_2$ Methoxy ist, die anderen von $R_1$, $R_2$ und $R_3$ nicht gleichzeitig Wasserstoff sein können, sowie pharmazeutisch annehmbare Salze davon.

2. Verbindung nach Anspruch 1, worin m 1 ist und n 1 ist.

3. Verbindung nach Anspruch 1, worin m 2 ist und n 1 ist.

4. Verbindung nach Anspruch 1, worin $R_1$, $R_2$ und $R_3$ aus der aus Wasserstoff, Hydroxy oder Methoxy bestehenden Gruppe genommen werden.

5. Verbindung nach Anspruch 1, worin $R_1$ und $R_2$ unter Bildung einer Methylendioxybrücke zusammengenommen sind.

6. Verbindung nach Anspruch 1, worin $R_1$ und $R_2$ jeweils Hydroxy sind.

7. Verbindung nach Anspruch 3, worin $R_1$ und $R_2$ jeweils Hydroxy sind.

8. Verbindung nach Anspruch 7, worin $R_3$ Wasserstoff ist.

9. Verbindung nach Anspruch 1, worin $R_1$ und $R_3$ jeweils Methoxy sind.

10. Verbindung nach Anspruch 9, worin $R_2$ Wasserstoff ist.

11. Die Verbindung 5',6'-Dimethoxy-2-(1',2',3',4'-tetrahydro-1'-naphthyl)pyrimidin und ihre pharmazeutisch annehmbaren Salze.

12. Die Verbindung 5',6'-Dihydroxy-2-(1',2',3',4'-tetrahydro-1'-naphthyl)pyrimidin und ihre pharmazeutisch annehmbaren Salze.